# EUROPÄISCHE PATENTANMELDUNG

(11) **EP 2 853 632 A1**
(43) Veröffentlichungstag der Anmeldung: **01.04.2015**
(21) Anmeldenummer: 13186209.6
(22) Anmeldetag: 26.09.2013
(51) Int. Cl.: D06F 39/02, C11D 3/386, C11D 7/32, C11D 7/50

(54) **Kit und Verfahren zur Reinigung und Desinfektion von medizinischen Instrumenten und Apparaten**

(71) Anmelder: Chemische Fabrik Dr. Weigert GmbH & Co. KG, 20539 Hamburg (DE)
(72) Erfinder: Strodtholz, Iris, 20539 Hamburg (DE); Reeßing, Petra, 20539 Hamburg (DE); Staffeldt, Jürgen, 21423 Winsen (DE)
(74) Vertreter: Glawe, Delfs, Moll

(57) **Zusammenfassung**

Gegenstand der Erfindung ist ein Kit zur maschinellen Reinigung und/oder Desinfektion medizinischer und/oder chirurgischer Instrumente und/oder Apparate, der eine erste Komponente, die wenigstens 15 Gew.-% Alkanolamin und wenigstens 10 Gew.-% Komplexbildner enthält, und eine zweite Komponente mit wenigstens einem Enzym enthält. Dieser Kit ermöglicht ein wirksames Reinigen und Desinfizieren chirurgischer und medizinischer Instrumente mit sehr geringen Einsatzmengen.

## Beschreibung

Die Erfindung betrifft einen Kit und ein Verfahren zur maschinellen Reinigung und/oder Desinfektion medizinischer und/oder chirurgischer Instrumente und/oder Apparate.

Chirurgische Instrumente sowie andere medizinische Geräte werden im Krankenhaus üblicherweise unter Verwendung alkalischer Reinigungsmittel maschinell gereinigt und anschließend chemisch oder thermisch desinfiziert. Solche stark alkalischen Mittel können aggressiv gegenüber empfindlichen Oberflächen wirken. Mit Blut verunreinigte chirurgische Instrumente werden häufig unmittelbar nach ihrer Benutzung bspw. in eine Desinfektionslösung abgelegt und verbleiben darin zunächst, bis sie zum Reinigen in die Spülmaschine geräumt werden. Das Blut wird durch die Desinfektionsmittel koaguliert und die im Blut enthaltenen Eiweißbestandteile durch den Desinfektionswirkstoff denaturiert. Solche besonders hartnäckigen Blutrückstände sind häufig nur durch alkalisch-aktivchlorhaltige Reinigungsmittel zu entfernen. Die oxidierende Aktivchlorkomponente bewirkt die Zersetzung der denaturierten Eiweißbestandteile. Auch andere Bestandteile von Desinfektionsmitteln wie bspw. Jod können schwer zu entfernende Rückstände bilden.

Die alkalisch-aktivchlorhaltigen Reiniger weisen die Nachteile auf, dass sie kennzeichnungspflichtige Gefahrenstoffe enthalten, dass bei ihrer Handhabung besondere Vorsichtsmaßnahmen zum Schutz des Bedienungspersonals erforderlich sind und daß sie im Abwasser eine unerwünschte Umweltbelastung darstellen.

Aus US-A-4 243 546, EP-A-0 481 663 und EP-A-0 730 024 sind enzymhaltige Reinigungsmittel bekannt, die insbesondere Blutproteine enzymatisch abbauen können.

EP-A-1 021 519 offenbart ein enzymfreies Reinigungsmittelkonzentrat zur Reinigung chirurgischer Instrumente. Als Nachteil der enzymatischen Reiniger wird dort genannt, dass die Enzymaktivität bei längerer Lagerung nachlassen kann.

EP-A-1 327 674 offenbart ein Reinigungsmittelkonzentrat für medizinische Instrumente, das ein Alkanolamin, Enzyme und Aminopolycarbonsäuren als Komplexbildner enthält.

Der Erfindung liegt die Aufgabe zugrunde, eine Möglichkeit zur wirksamen Reinigung medizinischer und chirurgischer Instrumente und Apparate zu schaffen, die in der Praxis einfach handhabbar ist und bei der lediglich geringe Volumina von Reinigungsmitteln vorzuhalten sind.

Die Erfindung löst diese Aufgabe durch einen Kit zur maschinellen Reinigung und/oder Desinfektion medizinischer und/oder chirurgischer Instrumente und/oder Apparate, dadurch gekennzeichnet, dass er enthält:
a) eine erste Komponente, die wenigstens 15 Gew.-% Alkanolamin und wenigstens 10 Gew.-% Komplexbildner enthält;
b) eine zweite Komponente, die wenigstens ein Enzym enthält.

Zunächst seien einige im Rahmen der Erfindung verwendete Begriffe erläutert.

Reinigungs- und/oder Desinfektionsmittel für medizinische und/oder chirurgische Elemente und Apparate sind solche Mittel, die im Zuge der Aufarbeitung solcher Instrumente die Schmutz- und Keimbelastung zumindest verringern.

Eine maschinelle Reinigung erfolgt in einer geeigneten Spülmaschine, vorzugsweise in einem üblichen Reinigung- und Desinfektionsgerät für medizinische bzw. chirurgische Instrumente (RDG). Dieser Begriff ist definiert in ISO 15883-1.

Der erfindungsgemäße Kit enthält wenigstens zwei Komponenten. Die erste Komponente (auch Alkalikomponente genannt) enthält verhältnismäßig hohe Konzentrationen Alkanolamin und Komplexbildner. Die zweite Komponente (auch Enzymkomponente genannt) enthält wenigstens ein Enzym, vorzugsweise ein proteolytisches Enzym. Die Menge dieser proteolytischen Enzyme liegt bevorzugt zwischen 0,05 und 1 Anson-Einheiten pro g Komponente.

Überraschenderweise hat sich gezeigt, dass sich durch Aufteilen dieser grundsätzlich bekannten Bestandteile eines Reinigungsmittels auf zwei Komponenten in der beanspruchten Art und Weise eine sehr wirksame Reinigung bzw. Desinfektion medizinischer Instrumente mit sehr geringen Wirkstoffmengen und vor allen Dingen auch mit sehr geringen eingesetzten Volumina der beiden Komponenten erreichen lässt.

Wie unten in den Beispielen noch erläutert werden wird, kann beispielsweise eine im Volumenverhältnis eins zu eins hergestellte Mischung von zwei erfindungsgemäßen Komponenten des Kits als 0,2 %ige wässrige Lösung angesetzt werden und hat eine gute Wirksamkeit gegenüber den üblichen Mischverschmutzungen chirurgischer Instrumente.

Erfindungsgemäß ist es somit möglich, im Krankenhausbetrieb die Komponenten des Kits in Vorratsbehältern mit einem Inhalt von beispielsweise 5 bis 20 l zu bevorraten. Solche Behälter oder Kanister lassen sich leicht transportieren und können im Betrieb in unmittelbarer Nähe des verwendeten Reinigungs- und Desinfektionsgerätes gelagert und angeschlossen werden. Es ist erfindungsgemäß nicht erforderlich, für eine hinreichende Betriebsdauer große Volumina beispielsweise in Form von Fässern mit 200 l Inhalt zu bevorraten, die in der Regel ein aufwändiges System von Versorgungsleitungen benötigen, da Fässer mit einem solchen Inhalt üblicherweise nicht unmittelbar am Reinigungs- und Desinfektionsgerät gelagert werden können, sondern in einem separaten Lagerraum angeordnet werden und dementsprechend über lange Leitungen mit der Verbrauchsstelle verbunden werden müssen.

Überraschenderweise hat sich gezeigt, dass der Einsatz eines Alkanolamins als Bestandteil der alkalischen Komponente in der beanspruchten Mindestkonzentration zu einer besonders guten Wirksamkeit bei sehr geringen Dosiermengen bzw. Konzentrationen der Komponenten des Kits in die wässrige Reinigungslösung führen. Die erfindungsgemäß vorgesehene Auftrennung der Bestandteile des Reinigungs- bzw. Desinfektionsmittels auf zwei Komponenten erlaubt das Bereitstellen so genannter Hochkonzentrate, bei denen nicht oder nur

schwer miteinander kompatible Inhaltsstoffe (beispielsweise alkalische Alkanolamine einerseits und Enzym andererseits) in höheren Konzentrationen stabil gelagert und vorrätig gehalten werden können als bei einem Reinigungsmittel mit lediglich einer Komponente.

Der Alkanolamingehalt der ersten Komponente beträgt bei einer bevorzugten Ausführungsform der Erfindung 15-30 Gew.-%, weiter vorzugweise 15-25 Gew.-%. Das Alkanolamin kann bevorzugt die folgende Struktur aufweisen: 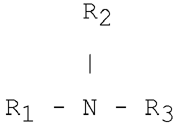 wobei R₁ eine Hydroxyalkylgruppe mit 1 bis 6 C-Atomen darstellt und wobei R₂ und R₃ unabhängig voneinander die genannte Hydroxyalkylgruppe oder Wasserstoff darstellen.

Weiter bevorzugt ist das Alkanolamin ausgewählt aus der Gruppe bestehend aus Mono-, Di- und/oder Triethanolamin.

Die erste Komponente kann bevorzugt 10-30 Gew.-%, weiter vorzugsweise 15-25 Gew.-% Komplexbildner enthalten. Die Komplexbildner dienen der Wasserenthärtung und können durch Komplexieren von Erdalkalionen die Reinigungswirkung gegenüber OP-typischen Verunreinigungen verbessern. Bei den Komplexbildnern kann es sich um Homo-, Co- oder Terpolymere auf der Basis von Acrylsäure oder deren Alkalisalzen handeln, ferner um Phosphonsäuren bzw. deren Alkalisalze, wie bspw. 1-Hydroxyethan-1,1-diphosphonsäure, Aminotrismethylenphosphonsäure, Ethylendiaminotetrakismethylenphosphonsäure, Phosphonobutantricarbonsäure; Weinsäure, Citronensäure und Glukonsäure; ferner Nitrilotriessigsäure oder Ethylendiaminotetraessigsäure bzw. deren Salze. Bevorzugt ist die Verwendung von Chelatbildnern, insbesondere Amino(poly)carbonsäuren sowie deren Salzen, beispielsweise das Trinatriumsalz der Methylglycindiessigsäure (MGDA).

Die korrosive Wirkung von Komplexbildnern auf eloxierte Aluminiumoberflächen und dergleichen kann vermindert bzw. vollständig vermieden werden durch den Zusatz wenigstens eines Mono- und/oder Diesters der Phosphorsäure mit aliphatischen Alkolholen der Kettenlänge C₁ bis C₂₂ und/oder aliphatischen Diolen und/oder aliphatischen Polyolen der Kettenlänge C₂ bis C₂₂. Besonders bevorzugt ist ein Diester der Phosphorsäure mit Butanol einerseits und Ethylenglykol andererseits. Dieser Ester ist kommerziell unter der Bezeichnung Hordaphos® MDGB erhältlich. Erfindungsgemäß erhält man so eine gute Reinigungswirkung auch bei Verwendung harten Wassers und trotzdem eine schonende Einwirkung auf eloxierte Aluminiumflächen.

Die zweite Komponente kann wenigstens ein proteolytisches Enzym enthalten. Geeignete proteolytische Enzyme sind bspw. von der Fa. Novozymes unter der Bezeichnung Esperase® 8.0 L, Subtilisin® oder Liquanase® Ultra 2.0 L erhältlich.

Die zweite Komponente kann erfindungsgemäß Tenside, vorzugsweise nichtionische Tenside, enthalten. Der Zusatz anionischer und kationischer Tenside ist ebenfalls möglich.

Die nichtionischen Tenside können erfindungsgemäß ausgewählt sein aus der Gruppe bestehend aus Fettalkoholethoxylaten, Fettalkoholpropoxylaten, EO-PO-Blockcopolymeren, Alkylglucosiden, Alkylpolyglucosiden, Oktylphenolethoxylaten und Nonylphenolethoxylaten. Dem Fachmann geläufige ethoxylierte und/oder propoxylierte Fettalkohole sind besonders bevorzugt. Solche nichtionischen Tenside sind beispielsweise von den Firmen Cognis oder Julius Hoesch erhältlich.

Insbesondere der zweiten Komponente können übliche Konservierungsmittel zugesetzt werden, bspw. p-Hydroxybenzoesäure oder deren Methylester, 5-Brom-5-Nitro-1,3-dioxan, Salicylsäure, 2-Naphtyl-m-N-Dimethylthiocarbanilat, 5-Chlor-5-methyl-4-isothiazolin-3-on, 2-Methyl-4-isothiazolin-3-on, Cetyltrimethylammoniumchlorid (CTAC) sowie Gemische dieser Verbindungen. Ein weiteres Konservierungsmittel ist p-Hydroxybenzoesäure bzw. deren Methylester. Mit Hilfe dieser Konservierungsmittel läßt sich Mikroben- und Pilzbefall der Komponenten vermeiden.

Bei Bedarf können Konfektionierhilfsmittel (Lösungsvermittler) zugegeben werden wie bspw. Natriumcumolsulfonat, Natriumtoluolsulfonat, Natriumxylolsulfonat, Harnstoff, Glykole, insbesondere Polypropylenglykole und Polyethylenglykole, Methylacetamid und Fettalkohole, wie bzw. Cetylalkohol.

Die Aufzählung möglicher Inhaltsstoffe ist nicht abschließend. Es können zusätzlich bspw. Netzmittel, Emulgatoren, schaumbremsende Mittel oder dergleichen zugesetzt werden. Vorteilhaft ist beispielsweise der Zusatz von N-Acylglutamat als Netzmittel.

Ein weiterer Gegenstand der Erfindung ist die Verwendung eines erfindungsgemäßen Kits zur maschinellen Reinigung und/oder Desinfektion medizinischer und/oder chirurgischer Instrumente und/oder Apparate.

Eine solche maschinelle Reinigung erfolgt ohne menschliches Zutun während eines automatischen Programmablaufs, insbesondere in einer üblichen Instrumentenspülmaschine (RDG). Mit dem Merkmal "Reinigung und/oder Desinfektion" soll zum Ausdruck gebracht werden, dass der erfindungsgemäße Kit sowohl bei der Kombination von Reinigung und Desinfektion in einem einzigen Verfahrensschritt als auch bei Programmabläufen verwendet werden kann, bei denen einem Reinigungsschritt ein separater Desinfektionsschritt nachfolgt. In diesem Fall ist der erfindungsgemäße Kit in beiden dieser Schritte verwendbar.

Ebenfalls Gegenstand der Erfindung ist ein Verfahren zum Reinigen von medizinischen und/oder chirurgischen Instrumenten und/oder Apparaten, gekennzeichnet durch folgende Schritte:
a) Aufbringen einer 0,05 bis 0,5%igen wässrigen Lösung der Komponenten eines erfindungsgemäßen Kits,
b) Einwirkenlassen der Lösung bei einer Temperatur von Raumtemperatur bis zur Siedetemperatur der Lösung,
c) Nachspülen.

Bei dem erfindungsgemäßen Verfahren geschieht das Aufbringen der wässrigen Lösung des Reinigungsmittelkonzentrats vorzugsweise durch Sprühen, kann aber bspw. auch durch Eintauchen oder Begießen erfolgen. Die Prozentangaben sind Gewichtsprozente, sofern im konkreten Einzelfall nicht anders angegeben.

Das Einwirkenlassen kann nach dem Aufbringen der Reinigungslösung ein Einwirken in Ruhe, d.h. ohne fortlaufendes Aufbringen bzw. Aufsprühen oder mechanisches Umwälzen bzw. Bewegen der Reinigungslösung umfassen. Bevorzugt ist im Zuge des maschinellen Reinigens bzw. Desinfizierens ein ständiges oder unterbrochenes Umwälzen der Reinigungslösung in dem verwendeten RDG, dabei wird die Lösung bevorzugt fortlaufend auf die Apparate bzw. Instrumente aufgesprüht bzw. aufgespült.

Die Komponenten des Kits können unmittelbar in die als Reinigungsflotte verwendete wässrige Lösung eindosiert werden.

Das Einwirkenlassen in Schritt b) erfolgt bevorzugt bei Raumtemperatur bis 55°C, vorzugsweise bei 35 bis 50°C, weiter vorzugsweise bei 40 bis 50°C.

Die wässrige Lösung der Komponenten des Kits weist bevorzugt einen pH-Wert von 9 bis 11, weiter vorzugsweise 10 bis 11 auf. Sofern im Rahmen der vorliegenden Anmeldung pH-Werte einer verdünnten Lösung des Reinigungsmittelkonzentrats gemessen werden, wird als Lösungsmittel vollentsalztes Wasser (VE-Wasser) verwendet. Wenn das Konzentrat mit üblichem Leitungswasser zu einer gebrauchsfertigen Lösung angesetzt wird, können sich je nach Beschaffenheit dieses Wassers geringfügig abweichende pH-Werte ergeben.

Die Einstellung des pH-Werts der Konzentrate/Komponenten auf den gewünschten Bereich erfolgt ggf. vorzugsweise durch Zusatz von Säuren und/oder geeigneter Puffersysteme. Bevorzugt ist der Zusatz von wenigstens einer organischen Säure ausgewählt aus der Gruppe bestehend aus Mono-, Di- oder Tricarbonsäuren mit 2 bis 6 C-Atomen. Unter diesen Säuren sind bevorzugt Citronensäure, Weinsäure, Äpfelsäure, Milchsäure, Glykolsäure, Glyoxylsäure, Bernsteinsäure, Adipinsäure und Glutarsäure. Citronensäure ist besonders bevorzugt. Die Säuren werden dem Konzentrat vorzugsweise in geringen Mengen zugesetzt. Einige dieser genannten organischen Säuren wie beispielsweise Citronensäure sind gleichzeitig Komplexbildner im Sinne der Erfindung.

Die Einwirkzeit in Schritt b) beträgt bevorzugt 1 min bis 30 min, weiter vorzugsweise 3 bis 20 min, weiter vorzugsweise 5 bis 15 min.

Im Rahmen des erfindungsgemäßen Verfahrens ist es möglich, das vor Beginn des Reinigungsvorgangs der Typ der medizinischen Instrumente und Apparate durch geeignete technische Maßnahmen erkannt wird und abhängig von diesem Typ ein geeignetes Reinigungs- und Desinfektionsverfahren ausgewählt wird. Die Reinigungs- und Desinfektionsgeräte für das erfindungsgemäße Verfahren können erfindungsgemäß eine Programmsteuerung zur Durchführung einer Mehrzahl unterschiedlicher Reinigungs- und Desinfektionsverfahren aufweisen. Diese Programmsteuerung ist zur bedarfsgerechten Dosierung der beiden Komponenten abhängig von dem durchgeführten Reinigungs- und Desinfektionsverfahren ausgebildet. Die Programme können bevorzugt unterschiedliche Konzentrationen der beiden Komponenten aufweisen und auch das Verhältnis zueinander der verwendeten Komponenten unterschiedlich einstellen. Beispielsweise kann bei der Aufbereitung herkömmlicher chirurgischer Instrumente eine höhere Konzentration der Alkalikomponente gewählt werden als bei der Aufbereitung empfindlicher Instrumente für die minimalinvasive Chirurgie (MIC-Instrumente) oder von Endoskopen. Das Volumenverhältnis der beiden Komponenten zueinander kann erfindungsgemäß bevorzugt zwischen 1:10 und 10:1, weiter vorzugsweise 1:5 und 5:1, weiter vorzugsweise 1:2 und 2:1 liegen. Bei einer Standardreinigung bzw. -desinfektion kann es beispielsweise 1:1 betragen. Die Konzentration beider Komponenten zusammen in der Reinigungslösung liegt beim erfindungsgemäßen Verfahren zwischen 0,05 und 0,5 Gew.-%, vorzugsweise 0,08 und 0,3 Gew.-%. Bevorzugte Bereiche für jede einzelne der beiden Komponenten sind 0,02 bis 0,3 Gew.-%, weiter vorzugsweise 0,04 und 0,15 Gew.-%.

Die unterschiedlichen Programme und die dabei jeweils eingesetzten Komponenten sowie deren Konzentrationen und Konzentrationsverhältnisse können als zugelassene Prozesse zur Aufbereitung von Medizinprodukten validiert werden. Es ist dann eine einfache und vollständige Dokumentation aller Aufbereitungsvorgänge einschließlich Art, Menge und gegebenenfalls auch Chargennummer der verwendeten Komponenten möglich.

Erfindungsgemäß kann vorgesehen sein, dass eine Einrichtung zur Erkennung des Typs der zu reinigenden und desinfizierenden medizinischen Instrumente und Apparate und zur Auswahl eines geeigneten Reinigungs- und Desinfektionsverfahrens bzw. -Programms abhängig von diesem Typ vorgesehen ist. Diese Vorgehensweise kann insbesondere dann sinnvoll sein, wenn jeweils Chargen identischer oder ähnlicher Instrumente gereinigt werden, beispielsweise übliche chirurgische Instrumente, MIC-Instrumente oder dergleichen. Die Erkennung des Typs der Instrumente kann mechanisch oder optisch erfolgen, beispielsweise durch Abtasten der Höhe im Instrumentenkorb, optische Erkennung der Instrumente oder dergleichen. Bevorzugt ist es, wenn entweder an den Instrumenten selbst oder an den Körben, mittels denen diese in das Reinigungs- und Desinfektionsgerät verbracht werden, Codiereinrichtungen vorhanden sind, die mit entsprechenden Leseeinrichtungen des Reinigungs- und Desinfektionsgerätes zusammenwirken. Beispielsweise kann es sich hierbei um RFID-Tags und RFID-Leseeinrichtungen handeln.

Bei dem erfindungsgemäßen Verfahren kann der Typ der Instrumente und Apparate ausgewählt sein aus der Gruppe bestehend aus konventionellen chirurgischen Instrumenten, Instrumenten für die minimalinvasive Chirurgie, Endoskopen und deren Teilen, Instrumenten für die Neurochirurgie, Instrumenten für die Augenchirurgie, Anästhesie-Utensilien, Container für medizinischen Instrumente und Apparate, und OP-Schuhen.

Die Erfindung wird nachfolgend anhand von Ausführungsbeispielen erläutert.

### Beispiel 1

Eine erfindungsgemäße Alkalikomponente wird anhand der Angaben der nachfolgenden Tabelle zubereitet. Die Mengen der einzusetzenden Ausgangsstoffe sind in Gewichtsteilen angegeben.

| | |
|---|---|
| Triethanolamin 99% | 20,0 |
| MDGB¹ | 2,0 |
| KOH, 45% | 3,0 |
| MGDA 3 Na, 40% | 40,0 |
| Acumer^{®} 2000² | 3,5 |
| Wasser (vollentsalzt) | 31,5 |
| pH-Wert des Konzentrats | 11,8 |
| pH-Wert einer 0,1%igen wässrigen Lösung (in VE-Wasser) | 10,8 |

| | |
|---|---|
| ¹ Diester der Phosphorsäure mit Butanol und Ethylenglykol ² Komplexbildner auf Basis von Carboxylat/Sulfonat-Acrylcopolymeren, Fa. Rohm and Haas | |

Eine erfindungsgemäße Enzymkomponente wird wie folgt hergestellt:

| | |
|---|---|
| Na-Cumolsulfonat 40% | 5,0 |
| Subtilisin | 10,0 |
| FA C12/C14; 2EO/4PO³ | 0,8 |
| FA C12/C14; 5EO/4PO | 3,0 |
| FA C12/C15; 2EO/6PO | 0,1 |
| CTAC | 0,2 |
| Mischung Zitr./Äpfelsäure 40% | 0,28 |
| KOH, 45% | 0,02 |
| Wasser (vollentsalzt) | 80,6 |
| pH-Wert des Konzentrats | 11,8 |
| pH-Wert einer 0,1%igen wässrigen Lösung (in VE-Wasser) | 10,8 |

| | |
|---|---|
| ³ Ethoxylierte/propoxylierte Fettalkohole | |

### Beispiel 2

In diesem Beispiel wird die Reinigungsleistung der Erfindung geprüft.

Als Substrat für eine Testanschmutzung dienten raue DIN-Metallplättchen mit einer Größe von 1 x 9 cm.

10 ml heparinisiertes Hammelblut (Fa. Acila) werden mit 1 ml VE-Wasser gemischt und mit 150 µl Protaminsulfat (1.000 I.E./ml zur Inaktivierung von jeweils 1.000 I.E. Heparin) wieder gerinnungsfähig gemacht. Das so vorbereitete Blut wurde auf die Metallplättchen aufgetragen und über Nacht bei Raumtemperatur getrocknet.

In ein 1000 ml Becherglas wurden 900 ml Reinigungslösung gegeben. Diese Reinigungslösung enthielt erfindungsgemäß jeweils 0,1 Vol.-% der beiden Komponenten des Beispiels 1. In einem zweiten Becherglas enthielt die Reinigungslösung als Vergleichsbeispiel 1 Vol.-% des Konzentrats des Beispiels 1 der EP 1 327 674 A1 (Vergleichsbeispiel).

Die Reinigungslösungen wurden auf 55 °C erwärmt und mit einem Magnetrührer bei Stufe 3,5 gerührt. Die Metallplättchen wurden für 5 min in die Lösungen eingetaucht und danach mit VE-Wasser abgespült. Anschließend wurden die Metallplättchen über Nacht getrocknet, visuell beurteilt, danach mit 0,1 %iger Amidoschwarzlösung zur Prüfung auf verbleibende Restmengen von Proteinen eingefärbt und erneut visuell beurteilt.

Die erzielte Reinigungsleistung gemäß dem Beispiel der Erfindung war identisch wie die Reinigungsleistung des Vergleichsbeispiels. Dieses Beispiel belegt somit, dass sich mit wesentlich geringeren Konzentrationen von Reinigungsmitteln als im als Vergleichsbeispiel herangezogenen Stand der Technik und damit auch mit lediglich einem Bruchteil der eingesetzten Volumina von Reinigungskomponenten eine ebenso gute Reinigungsleistung erzielen lässt. Dies ist überraschend und aus dem Stand der Technik nicht ableitbar.

### Beispiel 3

Beim Reinigen bzw. Desinfizieren in einem RDG soll die Reinigungslösung möglichst effektiv umgepumpt werden. Behindert werden kann dieses Umpumpen durch unerwünschtes Schäumen der eingesetzten Tenside, dies führt dazu, dass der Pumpendruck sinkt und das Umwälzen beeinträchtigt wird.

Da die Komponenten des erfindungsgemäßen Kits in deutlich geringeren Konzentrationen eingesetzt werden als Reinigungsmittel des Standes der Technik, steht zu erwarten, dass dieses unerwünschte Schäumen in geringerem Maße als im Stand der Technik auftritt.

Um dies zu prüfen, wurde in einem RDG Miele PG 8536 der Umwälzpumpendruck gemessen beim Umwälzen der Reinigungslösung im Niedertemperaturbereich bei 30 °C. In diesem Temperaturbereich, der beim Aufheizen der Flotte durchlaufen wird, ist die Lösung besonders empfindlich gegenüber Schäumen.

Es zeigte sich, dass eine Lösung, die jeweils 0,1 Vol.-% der beiden Komponenten eines erfindungsgemäßen Kits enthält, einen nur sehr geringfügigen Abfall des Pumpendrucks zeigt, während eine Lösung gemäß dem Stand der Technik (1 Vol.-% des Beispiels 1 der EP 1 327 674 A1) einen deutlicheren Abfall des Pumpendrucks zeigte.

## Patentansprüche

1. Kit zur maschinellen Reinigung und/oder Desinfektion medizinischer und/oder chirurgischer Instrumente und/oder Apparate, **dadurch gekennzeichnet, dass** er enthält:
a) eine erste Komponente, die wenigstens 15 Gew.-% Alkanolamin und wenigstens 10 Gew.-% Komplexbildner enthält;
b) eine zweite Komponente, die wenigstens ein Enzym enthält.

2. Kit nach Anspruch 1, **dadurch gekennzeichnet, dass** der Alkanolamingehalt 15 bis 30 Gew.-%, vorzugsweise 15 bis 25 Gew.-% beträgt.

3. Kit nach einem der Ansprüche 1 bis 2, **dadurch gekennzeichnet, dass** das Alkanolamin folgende Struktur aufweist: 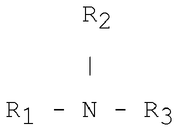 wobei R₁ eine Hydroxyalkylgruppe mit 1 bis 6 C-Atomen darstellt und wobei R₂ und R₃ unabhängig voneinander die genannte Hydroxyalkylgruppe oder Wasserstoff darstellen.

4. Kit nach Anspruch 3, **dadurch gekennzeichnet, dass** das Alkonolamin Mono-, Di- und/oder Triethanolamin umfasst.

5. Kit nach einem der Ansprüche 1 bis 4, **dadurch gekennzeichnet, dass** die erste Komponente 10 bis 30 Gew.-%, vorzugsweise 15 bis 25 Gew.-% Komplexbildner enthält.

6. Kit nach einem der Ansprüche 1 bis 5, **dadurch gekennzeichnet, dass** er als Komplexbildner Chelatbildner, vorzugsweise Chelatbildner ausgewählt aus der Gruppe bestehend aus Aminopolycarbonsäuren und deren Salzen, enthält.

7. Kit nach einem der Ansprüche 1 bis 6, **dadurch gekennzeichnet, dass** die zweite Komponente wenigstens ein proteolytisches Enzym enthält.

8. Kit nach einem der Ansprüche 1 bis 7, **dadurch gekennzeichnet, dass** die zweite Komponente Tenside, vorzugsweise nichtionische Tenside, enthält.

9. Verwendung eines Kits nach einem der Ansprüche 1 bis 8 zur maschinellen Reinigung und/oder Desinfektion medizinischer und/oder chirurgischer Instrumente und/oder Apparate.

10. Verfahren zum Reinigen von medizinischen und/oder chirurgischen Instrumenten und/oder Apparaten, **gekennzeichnet durch** folgende Schritte:
a) Aufbringen einer 0,05 bis 0,5%igen wässrigen Lösung der Komponenten eines Kits nach einem der Ansprüche 1 bis 8,
b) Einwirkenlassen der Lösung bei einer Temperatur von Raumtemperatur bis zur Siedetemperatur der Lösung,
c) Nachspülen.

11. Verfahren nach Anspruch 10, **dadurch gekennzeichnet, dass** das Einwirkenlassen in Schritt b) bei Raumtemperatur bis 55°C, vorzugsweise bei 35 bis 50°C, weiter vorzugsweise bei 40 bis 50°C, geschieht.

12. Verfahren nach Anspruch 10 oder 11, **dadurch gekennzeichnet, dass** die wässrige Lösung der Komponenten des Kits einen pH-Wert von 9 bis 11, vorzugsweise 10 bis 11 aufweist.

13. Verfahren nach einem der Ansprüche 10 bis 12, **dadurch gekennzeichnet, dass** die Einwirkzeit in Schritt b) 1 min bis 30 min, weiter vorzugsweise 3 bis 20 min, weiter vorzugsweise 5 s bis 15 min beträgt

14. Verfahren nach einem der Ansprüche 10 bis 13, **dadurch gekennzeichnet, dass** es zusätzlich die Schritte des Erkennens des Typs der medizinischen Instrumente und Apparate und der Auswahl eines geeigneten Reinigungs- und Desinfektionsverfahrens abhängig von diesem Typ umfasst.

15. Verfahren nach Anspruch 14, **dadurch gekennzeichnet, dass** der Typ der Instrumente und Apparate ausgewählt ist aus der Gruppe bestehend aus konventionellen chirurgischen Instrumenten, Instrumenten für die minimalinvasive Chirurgie, Endoskopen und deren Teilen, Instrumenten für die Neurochirurgie, Instrumenten für die Augenchirurgie, Anästhesie-Utensilien, Container für medizinischen Instrumente und Apparate, und OP-Schuhe.
